# EUROPEAN PATENT APPLICATION

(11) **EP 3 034 041 A1**
(43) Date of publication of application: **22.06.2016**
(21) Application number: 15200447.9
(22) Date of filing: 16.12.2015
(51) Int. Cl.: A61F 2/30, A61K 9/00, A61M 5/142, A61F 2/46

(54) **SYSTEMS FOR LOCAL ADMINISTRATION OF THERAPEUTICS**

(30) Priority: 16.12.2014 US 201462092389 P; 15.12.2015 US 201514969101
(71) Applicant: Oxford Performance Materials, Inc., South Windsor CT 06074 (US)
(72) Inventor: HACKING, Adam, Milton, MA 02186 (US); DEFELICE, Scott, Holyoke, MA 01040 (US); DECARMINE, Anthony, Lebanon, CT 06249 (US)
(74) Representative: Israelsson, Stefan

(57) **Abstract**

A load-bearing implant includes a body with a shape fabricated using selective laser sintering, the body being made of Polyetherketoneketone, at least one reservoir forming an internal cavity inside the body, the reservoir having an opening that provides fluid access to the internal cavity from outside the body, at least one fluid channel extending through the body between the at least one reservoir and an exterior surface of the body, and a fluid stored in the reservoir, the fluid facilitating osseointegration between the body and a bone. The at least one fluid channel communicates the fluid from the reservoir to the exterior surface of the body and provides a controlled rate of delivery of the fluid through at least one outlet in the exterior surface of the body.

## Description

### FIELD OF THE INVENTION

The present invention is directed to a system and method for implantation and local administration of therapeutics, and more specifically, to an implant delivering antibiotics, cells, antifungals and/or therapeutics to facilitate osseointegration, bone ongrowth and/or bone ingrowth.

### BACKGROUND OF THE INVENTION

A principal function of the skeletal system is to provide a load bearing framework for motion. The function of the skeleton can be impaired by injury, deformity or disease and can be restored by reconstruction with implanted devices. Ideally, the implanted device functions for the life of the patient.

Restoration of mobility, reduction in pain, and long-term problem-free function are the goals of skeletal reconstruction with implanted devices. A number of factors that influence long-term success have been identified. In general, it is desirable that materials and devices can withstand applied loads and transfer them to the surrounding bone tissue. It is also further desirable that the devices support and enable the attachment of bone tissue and prevent the formation of fibrous tissue. Imaging is routinely used to monitor tissue response, implant stability and/or progression of disease post-implantation; thus, it is desirable that the devices have minimal interference with medical imaging. The body is a corrosive environment, and many materials are subject to degradation. The by-products of such degradation can be harmful to the health of the patient.

Load-bearing capability is a function of both the material properties and the geometrical shape of the device. A mismatch in the stiffness between the implant (very stiff) and surrounding bone can lead to stress-shielding. Stress-shielding describes bone loss in the region adjacent to the implant. Peri-implant bone loss is a concern since it can result in the loss of implant fixation and the need for additional surgical treatment (revision surgery). Loss of implant fixation, pending loss of fixation or loss of bone around the implant complicates revision surgery.

A variety of materials have been used to manufacture long-term implanted load-bearing devices. Some desirable characteristics of implanted materials for skeletal reconstruction are high purity, appropriate stiffness, low cost, biocompatibility, radiolucency, ease of manufacture and the ability to support applied loads and the direct attachment of bone. Few materials possess all of these desirable attributes. For example, while stiff, many metals and ceramics used in skeletal reconstruction either block or distort the information obtained by imaging techniques such as computed tomography, magnetic resonance imaging and radiography. The release of ions from devices containing cobalt and chromium can cause toxicity and organ damage.

The preferred method for long-term stabilization of the implant is by the ongrowth or ingrowth of bone tissue. Ongrowth is defined as the micro-interlock of bone to the asperities of the implant surface at the nanometer and micrometer scales. Ingrowth is defined as the ingress of bone into the open, sub-millimeter sized spaces at the implant surface. Ongrowth or ingrowth of bone to an implant without an intervening fibrous tissue layer is referred to as osseointegration.

Osseointegration is a result of factors that include surface chemistry and morphology. Surfaces that are hydrophilic are desirable. Surfaces that also possess nanometer and micrometer sized features are also desirable. Osseointegration has been reported with a variety of titanium and hydroxyapatite coated implants but is generally not associated with polymers. For example, when placed in an osseous environment, the tissue response to polymeric materials like polyetheretherketone (PEEK) is generally fibrous. Approaches to modify the PEEK surface have been developed to improve tissue response. For example, coating the PEEK surface with titanium can enable the attachment of bone tissue and reduce the formation of fibrous tissue. Growth factors such as those from the TGF-B family, that includes BMP-2, stimulate bone formation. BMP-2 has been used to improve the tissue formation with PEEK implants.

Bone ingrowth is a fixation approach used with metallic implants. To enable bone ingrowth, implants can be coated with layers of beads or wire that create an open or porous surface. Preferred pore dimensions range from 0.5-0.8 mm and typical porosities range from 35-80%. An interconnected porosity is also preferred. Problems with porous coatings include debonding from the implant. In the case of titanium implants, the application of a porous coating can reduce the fatigue strength of the implant. Bulk porous materials, such as porous tantalum (also known as trabecular metal), enable bone ingrowth. Implants made with trabecular metal are expensive, heavy and interfere with imaging techniques.

All implanted devices are at risk of infection. This causes significant burden to the health care system. The burden of total joint infection (TJI) is estimated to cost $12 billion per year by 2015. The number of total joint replacement recipients in the US is rapidly growing from 650,000 annual replacements in 2010, to a predicted 3,500,000 per year in 2030. This growth is of great concern since the number of TJIs is proportional to the number of patients with total joint replacement and patients with TJI require prolonged hospitalization and consume a disproportionate amount of resources. Risk factors for TJI include obesity and diabetes, whose rise may increase the rate of TJI or post-operative complications. Of further concern is an increased risk of infection after total joint revision (7% to 23%) and an increased mortality rate (7-30%) at 5 years.

The treatment of TJIs by systemic ABx (antibiotics) alone is problematic since the local bone environment is poorly perfused and bacteria are protected by biofilms. When biofilms are present, as is often the case with chronic TJI, antimicrobial effects may require 1000 fold increase in ABx dose. For the past 40 years, the standard of care has been the addition of ABx to a polymer (poly-methyl methacrylate, PMMA, "bone cement") and implantation in the infection site. High levels of ABx elution from PMMA spacers achieve local therapeutic effects without systemic toxicity. However, there are limitations to this approach: the PMMA polymerization process is exothermic, so only a limited number of antibiotics are suitable for incorporation; ABx elution follows a burst release after which elution is variable and potentially suboptimal; antibiotics cannot be changed after spacer placement; spacers are not intended for permanent use, treatment takes many months, and in some cases, years. In addition, the use of static non-articulating spacers reduces mobility during treatment, which often leads to muscle wasting, reduced joint motion, and loss of soft tissue laxity.

An improved implant would enable imaging by MRI, CT and radiography, enable long term fixation by bone ongrowth and/or ingrowth, be made from a pure material that is resistant to corrosion, and possess a stiffness comparable to that of bone. An improved implant would also enable the delivery of therapeutics to treat infection, to enhance bone formation, to provide analgesia and/or to treat disease.

Recent manufacturing advances have enabled the generation of devices with desirable properties that provide improved function over currently used devices. Polyetherketoneketone (PEKK) is a high performance thermoplastic that can be fabricated into useful implantable devices by additive manufacturing techniques. PEKK devices are mechanically strong yet lightweight, radiolucent, capable of osseointegration and capable of therapeutic delivery.

### SUMMARY OF THE INVENTION

The needs set forth herein as well as further and other needs and advantages are addresses by the present embodiments, which illustrate solutions and advantages described below.

It is an object of the present teachings to provide an implant that promotes fixation by bone ongrowth and/or ingrowth and in particular, long-term fixation. It is an object of the present teachings to provide a method of making an implant which promotes long term fixation by bone ongrowth and/or ingrowth.

It is another object of the present teachings to provide an implant, and a method of making thereof, that enables attachment of bone tissue and prevents the formation of fibrous tissue or at least reduces the likelihood of fibrous tissue formation.

It is another object of the present teachings to provide an implant, and a method of making thereof, that promotes bone formation and provides treatment for infections and/or diseases.

It is another object of the present teachings to provide an implant, and a method of making thereof, that reduces imaging interference and enables medical imaging by MRI, CT, radiography, and/or the like.

It is another object of the present teachings to provide an implant, and a method of making thereof, that is resistant to or at least reduces the likelihood of corrosion.

It is also an object of the present teachings to provide an implant, and a method of making thereof, that has stiffness and load-bearing characteristics comparable to bone.

These and other objectives are achieved by a load-bearing implant which comprises a body with a shape fabricated using selective laser sintering, the body being made of Polyetherketoneketone (PEKK), and at least one reservoir forming an internal cavity inside the body. The reservoir has an opening that provides fluid access to the internal cavity from outside the body. The implant further includes at least one fluid channel extending through the body between the at least one reservoir and an exterior surface of the body. A fluid is stored inside the reservoir, wherein the fluid facilitates osseointegration between the body and a bone. The at least one fluid channel is configured to communicate the fluid from the reservoir to the exterior surface of the body and provides a controlled rate of delivery of the fluid through at least one outlet in the exterior surface of the body.

Other objectives are achieved by a load-bearing implant apparatus which comprises an implant and a fluid supply unit connected to the implant. The implant has a body with a shape fabricated using selective laser sintering, the body being made of Polyetherketoneketone (PEKK), and at least one reservoir forming an internal cavity inside the body. The reservoir has an opening that provides fluid access to the internal cavity from outside the body. The implant also includes at least one fluid channel extending through the body between the at least one reservoir and an exterior surface of the body. The implant contains a fluid stored inside the reservoir, wherein the fluid facilitates osseointegration between the body and a bone. The at least one fluid channel is configured to communicate the fluid from the reservoir to the exterior surface of the body and provides a controlled rate of delivery of the fluid through at least one outlet in the exterior surface of the body. Further, the fluid supply unit is connectable to the reservoir such that the fluid supply unit communicates the fluid to the reservoir through the opening.

According to one embodiment of the load-bearing implant defined in the annexed claim 2, said outlets and said fluid channels are positioned in rows along said body.

According to one embodiment of the load-bearing implant defined in the annexed claim 2, said outlets and fluid channels are divided into groups of one or more channels, said groups being arranged at different areas of said body.

According to one embodiment of the load-bearing implant defined in the annexed claim 10, said external supply is an intravenous bag containing said fluid.

According to one embodiment of the load-bearing implant defined in the annexed claim 1, said at least one fluid comprises a plurality of fluids that react with one another to provide synergistic effects for osseointegration.

Other features and aspects of the invention will become apparent from the following detailed description, taken in conjunction with the accompanying drawings, which illustrated by way of example the features in accordance with embodiments of the invention. The summary is not intended to limit the scope of the invention, which is defined solely by the claims attached thereto.

### BRIEF DESCRIPTION OF THE DRAWINGS

FIG. 1 is schematic illustrating a selective laser sintering process (herein "SLS"; also referred to as selective laser fusion) for making implant structures in accordance with the present teachings.
FIG. 2A - 2G shows different embodiments of femoral implants in accordance with the present teachings.
FIG. 2H shows a tool in accordance with the present teachings for removing particulate from channels formed in the implant structures using the SLS technique.
FIG. 2I - 2N shows other embodiments of femoral implants in accordance with the present teachings.
FIGS. 3A-3G illustrate an implant in accordance with the present teachings.
FIGS. 4-6 illustrate different views of an artificial acetabular cup in accordance with the present teachings.
FIGS. 7A-7D illustrate different views of a vertebral spacer in accordance with the present teachings.
FIGS. 8A-8C illustrate different views of a femoral component of an artificial knee replacement in accordance with the present teachings.
FIGS. 9A-9C illustrate different views of a tibial plateau of an artificial knee replacement in accordance with the present teachings.
FIG. 10 illustrates a model of an artificial knee spacer in accordance with the present teachings.
FIGS. 11-13 illustrate a system and method for fabricating small openings in implant structures made using the SLS technique in accordance with the present teachings.
FIGS. 14A-14F illustrate a system and method for fabricating small openings in the surface of an acetabular cup in accordance with the present teachings.

### DETAILED DESCRIPTION OF THE INVENTION

The present teachings are described more fully hereinafter with reference to the accompanying drawings, in which the present embodiments are shown. The following description is presented for illustrative purposes only and the present teachings should not be limited to these embodiments.

### A. SELECTIVE FUSION OF POLYMERS TO FORM AN IMPLANT.

Selective laser sintering ("SLS") is an additive manufacturing technique that uses electromagnetic radiation from a laser to fuse small particles of plastic, metal (direct metal laser sintering), ceramic, or glass powders into a mass that has a desired three dimensional shape. The laser selectively fuses powdered material by scanning cross-sections according to a three dimensional digital description of the part, also referred to as a build file, on the surface of a powder bed. After a cross section is scanned, the powder bed is lowered by one layer thickness, a new layer of material is applied, and the bed is rescanned. This process is repeated until the desired three dimensional shape part is completed. Although the term sintered is used in regard to the abbreviation SLS and the technique generally, it should be understood that in the context of polymer powders, such as PEKK discussed herein, the powder is fused together during the SLS process. Use of the term sinter or fuse is not intended to limit the present disclosure in anyway.

An SLS machine typically preheats the material in the powder bed. This is typically accomplished by preheating the actual bed, which transfers energy to the powder. The preheating of the powder makes it easier for the laser to raise the temperature of the selected regions of layer of unfused powder to the fusion point. When working with polymer powders in the SLS process, the bed temperature is set to a temperature specific to the polymer in use. This specified temperature is typically close to the melting point of the resin. The laser causes fusion of the powder only in locations specified by the input.

Laser energy exposure is typically selected based on the polymer in use and is between the amount required to fuse the resin and the amount that will cause degradation. Preheating of the material inhibits unwanted distortions in formed parts as they cool. After the layer-wise process is completed, the formed object(s) is in a cake of unfused powder, referred to as the cake. The formed object is extracted from the cake.

Polyaryletherketones ("PAEK") are of interest in the SLS process because parts that have been manufactured from PAEK powder or PAEK granulates are characterized by a low flammability, a good biocompatibility, and a high resistance against hydrolysis and radiation. The thermal resistance at elevated temperatures as well as the chemical resistance distinguishes PAEK powders from ordinary plastic powders. A PAEK polymer powder may be a powder from the group consisting of polyetheretherketone ("PEEK"), polyetherketone ketone ("PEKK"), polyetherketone ("PEK"), polyetheretherketoneketone ("PEEKK") or polyetherketoneetherketoneketone ("PEKEKK").

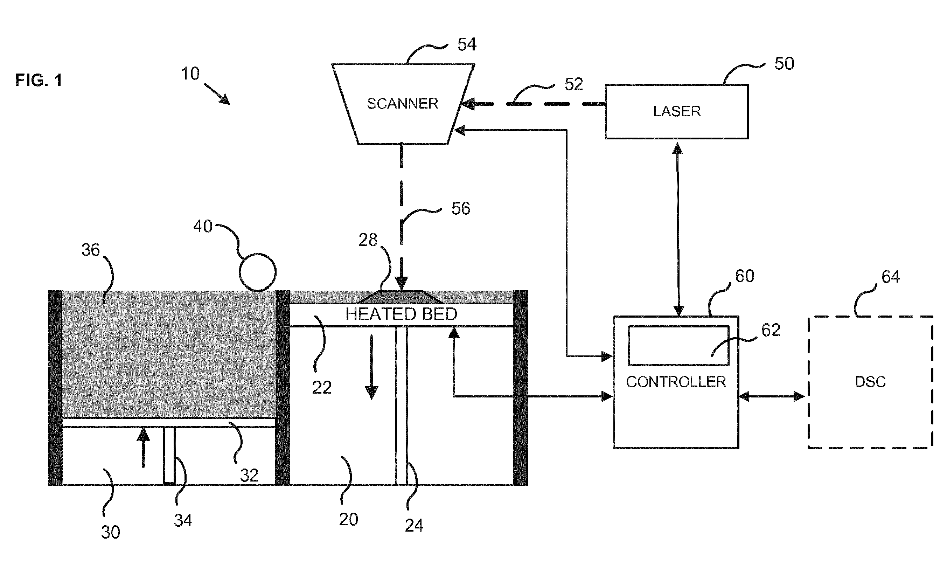

In reference to FIG. 1, an SLS machine 10 in accordance with the present teachings is illustrated. The system 10 includes a first chamber 20 having an actuatable piston 24 disposed therein. A bed 22 is disposed at an end of the piston 24. The temperature of the bed 22 can be variably controlled via a controller 60 in communication with heating elements in and or around the bed 22. The bed temperature setpoint is inputted through an interface 62 into the controller 60. Software executing on the controller 60 transmits a signal to one or more of the heating elements to heat the bed at or around the temperature setpoint. A second chamber 30 is adjacent to the first chamber 20. The second chamber 30 includes an actuable piston 34 and includes a table surface 32 disposed at an end of the piston 34 disposed therein. A powder 36 for use in the SLS machine 10 is stored in the second chamber 30 prior to the sintering step. In some embodiments, a digital signal control (DSC) unit 64 may be connected to the controller 60 to provide further control system functionalities to the controller 64, such as control algorithms and/or instructions. In other embodiments, the DSC unit 64 stores a build program which instructs the SLS machine 10 on how to construct an object (i.e., build 28). In alternative embodiments, the build program is stored in the controller 60 and/or is transferred from the DSC unit 64 to the controller 60.

During operation of the SLS machine 10, a spreader 40 translates across a top surface of the first chamber 20, evenly distributing a layer of powder 36 across either the top surface of the bed 22, or the material previously disposed on the bed. The SLS machine 10 preheats the powder material 36 disposed on the bed to a temperature proximate to a melting point of the powder. Preheating is typically accomplished by heating the actual bed, as described above, which transfers energy to the powder in the form of heat via thermal conduction. Preheating the powder makes it easier for the laser to raise the temperature of powder to a fusing point. In some embodiments of the present teachings, there may be heating elements in or around the second chamber 30 in order to heat the powder prior to being delivered to the sintering surface (e.g., bed 22).

A laser 50 and a scanning device 54 are disposed above the bed 22. The laser transmits a beam 52 to the scanner 54, which then distributes a laser beam 56 across the layer of powder 36 disposed on the bed 22 in accordance with a build program. The laser selectively fuses powdered material by scanning cross-sections generated from a three dimensional digital description of the part on the surface of bed having a layer of the powdered material disposed thereon. The laser 50 and the scanner 54 are in communication with the controller 60. After a cross-section is scanned, the bed 22 is lowered by one layer thickness, a new layer of powdered material is disposed on the bed via the spreader 40, and the bed is rescanned by the laser. This process is repeated until the build 28 is completed. During this process, the cylinder 34 in the second chamber 30 is incrementally raised to ensure that there is sufficient supply of powder.

It should be understood to a person of ordinary skill in the art and familiar with this disclose, that the present teachings are not limited to a particular type of SLS machine, or parts made from a particular SLS machine or process, and the above described configuration is only provided as a non-limiting example.

### B. PEKK IS ADVANTAGEOUS FOR USE IN IMPLANTS.

It has been discovered that polyetherketoneketone (PEKK) is advantageous for use as an implant. PEKK is radiolucent and light, has mechanical properties similar to bone, can be cut and drilled like bone and can be fabricated in complex geometries using the above described SLS methods.

In a recent study, the inventors demonstrated that native bone attached to implants made from PEKK. This result was unexpected because it was known in the art that bone apposition was typically very low for implants made from polymers, such as PEEK. The study assessed the bone and tissue response to PEKK implants surgically placed in an environment that supported bone formation and attachment. Two identical groups of rod like implants that differed only in surface chemistry were bilaterally implanted in the distal femora of a rabbit. The experimental implant group was a PEKK implant and the control group was a PEKK implant coated with a 150nm dense and homogenous layer of titanium dioxide. After 8 weeks, the femurs were harvested and processed for undecalcified thin section histology to determine the extent of bone apposition and tissue response.

Both implant groups showed evidence of bone attachment. Bone apposition to the control group (Ti coated PEKK, 26.92 % ± 12.80) was significantly higher than the experimental group (native PEKK, 15.84 % ± 12.56, p<0.0001). Basic histologic analysis did not indicate evidence of a fibrous or inflammatory response that was known to occur with PEEK implants. Although bone apposition was less for the native PEKK as compared to the Ti coated PEKK, the results of this study were exciting and unexpected because they demonstrated that unlike other polymers known in the art, bone attaches to PEKK in significant manner and without the fibrous or inflammatory response shown with PEEK implants.

Another advantage of implants made from PEKK is that they are radiolucent. As a result, implants made from PEKK do not block or distort the information obtained by imaging techniques such as computed tomography, magnetic resonance imaging and/or radiography. It is advantageous in using these imaging techniques to examine how the implant functions, but also to examine areas of the body above or below the implant. Although other materials, such as PEEK may be radiolucent, it has been shown that such materials have poor osteogenerative qualities. While it is known to apply a coating of titanium or other materials to the surface of such implants to enhance osteogeneration, such coatings typically render the implant radiopaque.

### C. IMPLANTS FOR DELIVERY OF THERAPEUTICS.

Both bone apposition and vascularization could be improved by endowing the materials with the capability of controlled and continued release of protein factors, such as BMPs or VEGF, growth factors. In addition, device-associated infections are a major problem with implanted materials. Methods and systems for providing controlled release of antibiotics, particularly those that could be loaded remotely after device insertion, may also be useful. Also, cells (Osteoblasts, tenocytes, fibroblasts, etc.), antifungals (some infections are fungal based), and broad therapeutics - analgesics, antibodies, genes, etc. may be helpful.

To this end, the inventors have developed new implants that comprise interior channels for delivering therapeutics to the interface between the implant and the surrounding structure. Generally speaking, the implants are made from PEKK using the SLS process described above. In some embodiments, the implants include a reservoir disposed therein. One or more of the channels are in fluid communication with the reservoir and an outer surface of the implant. In this manner, it is possible for a fluid in the reservoir to flow through the channels and to the interface between the bone and implant.

For example, the fluid may comprise therapeutics for enhancing bone growth and/or inhibiting infection, among other things. In other embodiments, the fluid may comprise elements for treating a disease, such as cancer. In one embodiment, the fluid may comprise a growth factor such as a bone morphogenetic protein (BMP), or a vascular endothelial growth factor (VEGF). In other embodiments, the fluid may comprise one or more antibiotics for inhibiting and/or treating infection. In some embodiments, as discussed below in reference to a knee spacer, it is possible to apply a negative pressure in the fluid communication system and withdraw bodily fluids through the system, thereby providing a tool for direct analysis of the tissue surrounding an implant. Accordingly, the fluid communication system can provide bidirectional fluid communication.

Therapeutic fluid(s) may be introduced to the implant, and particularly to one or more reservoirs and/or channels in the implant through a variety of different means. For example, in one embodiment, the implant is preloaded with a therapeutic fluid. After the implant is introduced into the patient, preloaded fluid in the fluid communication system flows through one or more channels to the interface between the tissue and implant. In such embodiments, the amount of therapeutic fluid is limited to the volume fluid initially preloaded in the system.

In other embodiments of the present teachings, the reservoir may be post-operatively recharged. For example, in one embodiment of the present teachings, an external supply of therapeutic fluid is in fluid communication with the reservoir via a catheter or the like. In this embodiment, the external supply of therapeutic fluid may be semi-permanently attached and in constant fluid communication with the reservoir. In yet other embodiments of the present teachings, an external supply of therapeutic fluid may be periodically connected to the catheter to provide fluid to the implant. In such embodiments, the schedule of connection may be prescribed, for example, in the course of recovery from a surgical procedure. Or, for example, external therapeutic fluid may be provided in response to an occurrence, for example, the onset of an infection, or a need for growth factor.

In some embodiments of the present teachings, the reservoir may be periodically refilled with a rigid needle which is inserted into the body of a patient so that it is in fluid communication with the reservoir of the implant. In other embodiments of the present teachings, an implant is provided with one or more channels for delivering therapeutic fluid, but is inserted into the patient without any such fluid. In such embodiments, therapeutic fluid is introduced into the implant, via a catheter, a needle, or some other means, only upon detection of a need for such therapy. For example, if a patient is diagnosed with an infection at the site of the implant, a fluid comprising an antibiotic fluid may be introduced into the reservoir. In some embodiments of the present teachings, the reservoir is connected to an IV bag to provide continuous fusion of therapeutics. In some embodiments of the present invention, a reservoir bag is implanted under the skin and is in fluid communication with a pump (e.g., implantable osmosis pump) for delivering the therapeutic. The reservoir bag may be refillable.

### D. DELIVERY OF THERAPEUTICS THROUGH IMPLANTS TO FACILITATE ONGROWTH.

Conventional textured or coated implant surfaces are designed to achieve bone-to-implant contact, which is referred to as ongrowth. In such implants, the drug eluting channels in accordance with the present invention are in fluid communication with the outer surface of the implant and the therapeutic fluid is eluted therefrom, thereby delivering therapeutic fluid to the interface between the implant and the bone. In some embodiments, the therapeutic fluid comprises a growth factor to facilitate growth of the desired tissue onto the surface of the implant. In some embodiments, it may be possible to vary the spatial relationship of such channels across the surface of the implant to provide sufficient therapeutic fluid across the entire interface. In such embodiments, it is possible to control the rate at which fluid is eluded from channels by varying, among other factors, the diameter of the channel openings, the diameter of the channels, the cross-sectional shape of the channels, the number of channels, the viscosity of the therapeutic fluid, the length of the channels, and the geometry of the interface. The implant may also be configured to deliver different fluids that have a reaction in the body or combine to have different or synergistic effects.

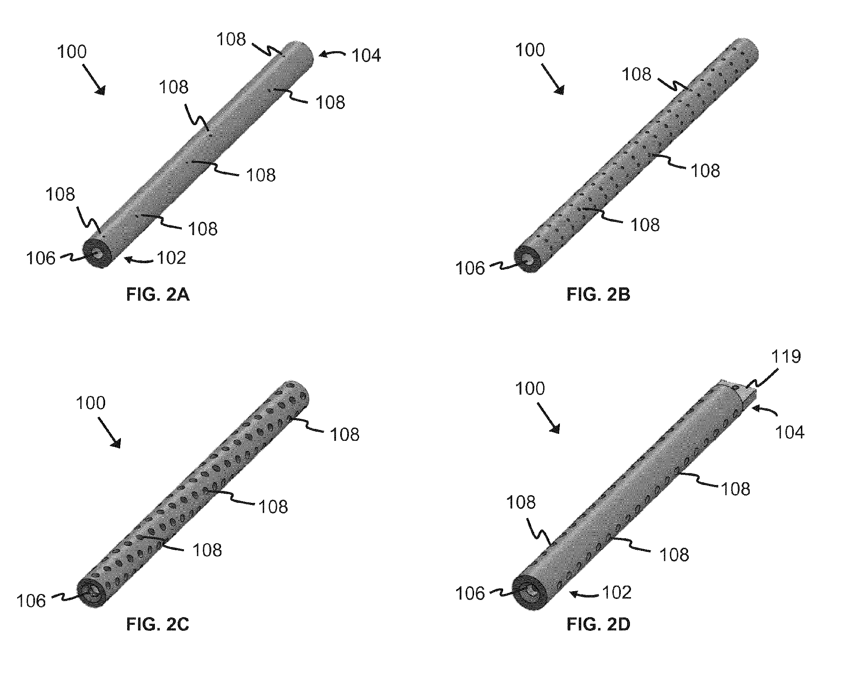

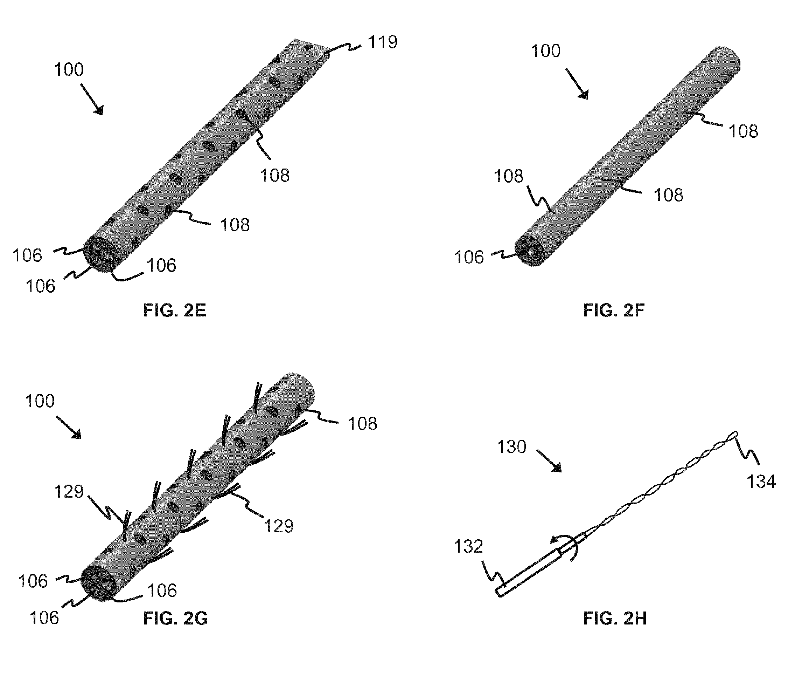

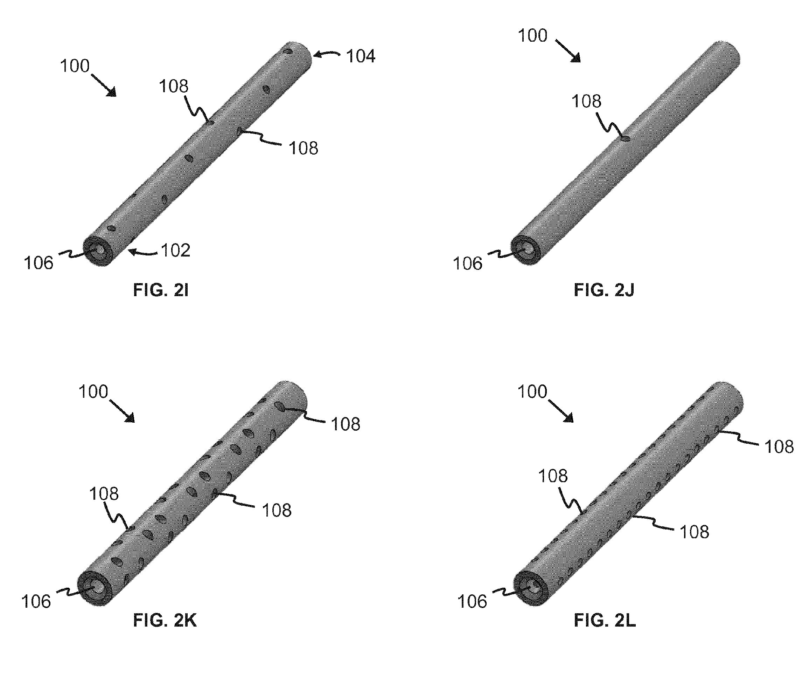

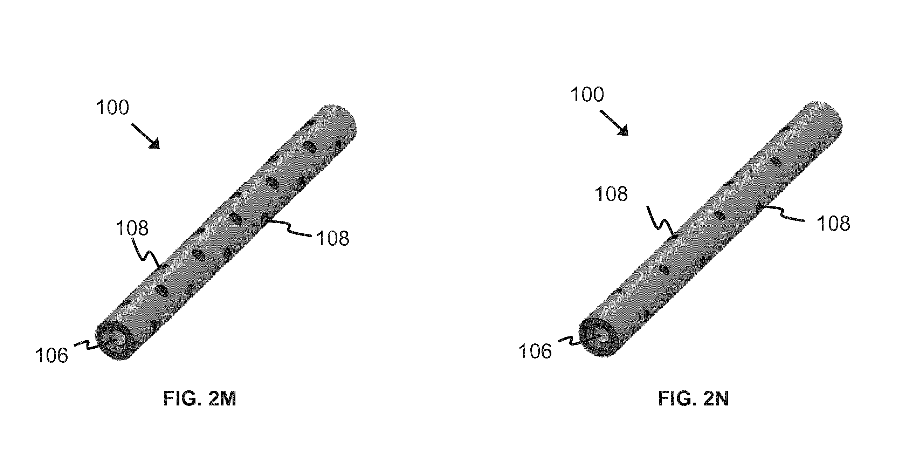

In reference to FIGS. 2A - 2G and 2J-2N, several embodiments of femoral rods are shown. The rods are made from PEKK using the SLS process described above. A person of ordinary skill in the art familiar with this disclosure will understand the geometry of the rod, for example, the length, the diameter, the shape, etc., may vary depending on a number of circumstances, including the size of the patient, the type of implant procedure, and the purpose of inserting the rod. As an example, the rod may be dimensioned with a length of 40.0mm, an outer diameter of 3.75mm, and an inner diameter of 1.21 mm, which are based on average sizes of New Zealand white rabbit femoral canals (used in testing). The size of the outer diameter (e.g., 3.75mm) is suitable for connection to an implantable osmosis pump.

The purpose of these figures is to illustrate different configurations of rods in accordance with the present teachings.

In reference to FIG. 2A, a rod 100 in accordance with the present teachings is shown. The rod 100 extends between a proximal end 102 and a distal end 104 along an axis. In the embodiment shown in FIG. 2A, the axis is longitudinal. It should be understood, however, that one advantage of using the SLS technique is that it enables the formation of curved channels that may be used in different implant applications. In the embodiment shown in FIG. 2A, the rod 100 has a substantially circular cross-section with a constant diameter along the axis thereof. It should be understood, however, that a rod in accordance with the present teachings may have different types of cross section and that such geometries may vary along the axis of the rod between the proximal end and the distal end.

In reference to FIG. 2A, the rod 100 defines a bore 106 that extends between the proximal end 102 and the distal end 104. In the embodiment shown in FIG. 2A, the bore 106 has a first opening at the proximal end 102 and/or a second opening at the distal end 104. It should be understood, however, that the present teachings are not limited in this regard and that the size, shape, configuration, and openings of the bore can be further configured to deliver therapeutic fluid in a different manner.

The rod 100 includes a plurality of channels 108. In the embodiment disclosed in FIG. 2A, each channel 108 is in fluid communication with the bore 106 and extends radially outward to an opening on the outer surface of the rod 100. In this manner, therapeutic fluid that is stored and/or received in the bore 106 can flow through the channels 108 to the interface between the rod and the surface of the medullary cavity in which the rod is received. The holes/openings of the channels 108 are configured with an arrangement where fluid flow through the rod would reach multiple sites throughout and along the rod. It is preferred that the rod 100 is in direct contact with the bone along the entire outer surface area of the rod 100 to facilitate ongrowth.

The rod 100 is made from PEKK using the above described selective laser sintering technique. The SLS technique enables construction of the unique fluid communication network in the rod 100 for delivery of therapeutic fluid. It should be understood, however, that the present teachings are not limited to a rod made from PEKK or made by the SLS technique, as other materials and fabrication techniques may be used to practice the presenting teachings. It should further be understood that the rod 100 is not limited to use in the femoral medullary cavity, and that it can be used in any cavity in the body.

FIGS. 2B-2G illustrate alternate embodiments of rods in accordance with the presenting teachings in which the number of bores and size thereof, and the number and placement of channels varies. These figures illustrate that the SLS technique can be used to fabricate a rod having varying fluid communication networks to address, for example, flow rate. FIG. 2B discloses an embodiment comprising a plurality of circumferential groupings of radially extending channels extending along the axis of the rod. FIG. 2C discloses an embodiment comprising a plurality of circumferential groupings of radially extending channels extending along the axis of the rod, wherein the openings have a greater diameter as compared to the embodiment shown in FIG. 2B, and wherein each successive axial circumferential grouping is rotated about the axis of the rod relative to the prior grouping.

In reference to FIG. 2D, another unique pattern of channels is disclosed. The rod further includes a block 119 proximate to its distal end. In some embodiments, the block 119 serves as a manufacturing aid, providing a point to hold the rod during post SLS cleaning and processing. In other embodiments, the block 119 provides a second opening to the bore that is perpendicular to the axis thereto. In reference to the embodiment shown in FIG. 2E, the rod comprises three bores that extend between the proximal end and the distal end thereof. FIG. 2E further includes one or more channels that are in fluid communication with the bore that do not extend directly radially inward, but also include a tangential component.

In reference to FIG. 2G, the rod comprises a plurality of barbs 129 extending radially out from the surface thereof and in the distal direction. The bards 129 inhibit movement of the rod relative to the bone after the rod has been inserted into the medullary cavity.

In reference to FIG. 2H, a tool 130 for removing unfused particles from the channel(s) and/or reservoir(s) is illustrated. As stated above, the layer wise building process of the SLS technique results in a cake bed of PEKK particles having the building parts disposed therein. Typically, the granulated powder is packed in the channels and the bores. This powder may be difficult to remove using traditional methods such as compressed air, fluid submersion, etc. The inventors have discovered the disclosed tool 130 for removing unfused powder. The tool 130 extends between the proximal end 102 and the distal end 104 of the rod 100. In the embodiment shown in FIG. 2H, the tool comprises a wire that it looped at its distal end 134. Two halves of the wire are twisted around each other in a helical pattern that extends to the proximal end 102. The wire is selected so that it is semi-flexible.

During use, the distal end 134 of the tool 130 is inserted into the opening of a bore 106 (or channel 108). The tool 130 is rotated about its axis, either by hand or by electric drill. This rotation loosens particles that are packed in the channels and bore. The helical configuration facilitates removal of the loosened particles from the bore 106 (or channel 108) as the tool is advanced further into the bore (or channel). The tool 130 and a method of using the same are particularly advantageous when the bore extends along a curved path. The flexibility of the wire allows the distal end 134 of the tool to be advanced down such a curved path, while the wire simultaneously retains the helical configuration, thereby enabling unfused particles to be loosened from curved channels.

FIGS. 2I-2N illustrate other embodiments of rods in accordance with the presenting teachings in which the number of bores and size thereof, and the number and placement of channels vary. In comparison to the rods shown in FIGS. 2A-2G, the rods of FIGS. 2I-2N are characterized with design parameters that are adjusted to limit the number of channels along the length of the rod, to limit the total number of holes throughout the implant depending on their size and whether they are in the same cross-sectional plane perpendicular to the longitudinal axis of the rod, and to limit the hole diameters to between 0.75mm and 1.00mm, for example. The designs shown in FIGS. 2I-2N have been tested and optimized via computational fluid dynamic modeling to deliver a desired concentration of antibiotic to bone surrounding the implant.

### E. IMPLANTS TO FACILITATE INGROWTH AND SYSTEMS AND METHODS FOR DELIVERING THERAPEUTIC THROUGH THE SAME.

In some circumstances, bone ingrowth, as opposed to bone ongrowth, is preferred. For example, during spinal surgery, bone ingrowth is sought between the implant in the surrounding disks to provide for increased strength. Bone ingrowth is defined as the ingress of bone into the open, sub-millimeter sized spaces at the implant surface.

The inventors have discovered new geometries for implants that encourage bone ingrowth and that can be fabricated from PEKK using the SLS technique. The inventors have also applied the above described systems and methods for delivering therapeutic fluids to such new geometries. Although some of the applications may be described together below, it is not intended to limit the present disclosure, and these technologies may be used by themselves.

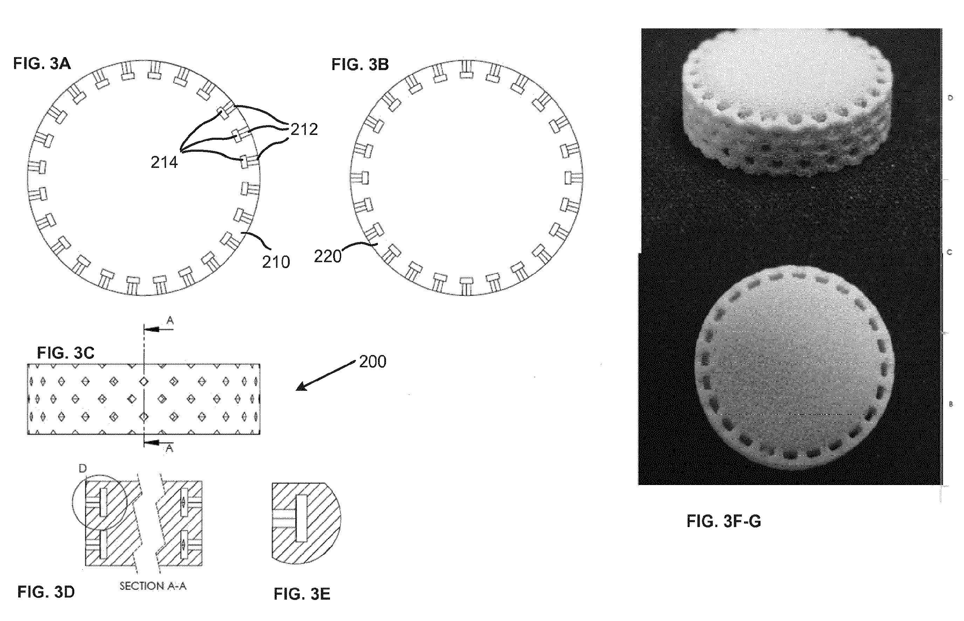

In reference to FIGS. 3A-G, a sample showing structures in accordance with the presenting teachings to facilitate bone ingrowth is illustrated. The structure comprises a disc 200 made from PEKK using the SLS process. It should understood, however, the present teachings are not limited to PEKK or the SLS technique, as other materials and fabrication techniques may be used to practice the presenting teachings.

FIG. 3A shows a first layer 210 of the disc 200. FIG. 3B shows a second layer 220 of the disc 200. FIG. 3C shows a side view of the disc 200. FIG. 3D shows a cross section A-A of the disc 200 in FIG. 3C. FIG. 3E shows a detail D of the disc 200 in FIG. 3D. Further, FIG. 3F illustrates a perspective view of the disc 200, and FIG. 3G illustrates a top view of the disc 200.

In reference to FIGS. 3A, the first layer 210 of the disc 200 is generally cylindrical, having a circular cross section and a uniform height. The first layer 210 comprises a plurality of diamond shaped channels 212. Each channel 212 extends radially inward from a diamond shaped opening in the surface of the disc 200. Each diamond shaped channel 212 extends inward and is in fluid communication with a reservoir 214. The reservoir 214 comprises a rectilinear cavity that extends vertically through the first layer 210. The plurality of diamond shaped channels 212 and associated reservoirs 214 are equally spaced along the circumference of the first layer 210.

The second layer 220 comprises an identical pattern of channels and reservoirs. The first layer 210 and the second layer 220 are joined together; however, the second layer 220 is rotated relative to the first layer 210 about the vertical axis of the disc 200. Through this rotation, the reservoirs of the first layer 210 are slightly offset from the reservoirs of the second layer 220. This configuration enables fluid communication between reservoirs in the first layer and second layer, while simultaneously increasing material connection therebetween, thereby increasing the strength of the structure. With this arrangement, it is possible to provide a structurally sound implant which promotes bone ingrowth by way of the diamond shaped channels and still enables robust fluid communication on the interior layer for delivering therapeutics to ingrowth in the channels. This pattern repeats itself though the structure.

It should be understood that while a disc configuration is shown, a person of ordinary skill in the art and familiar with this disclosure will understand that the present teachings are not limited in this regard, and that the inventive pattern combining a porous ingrowth layer and an adjacent fluid delivery layer can manifest itself in many different forms and geometries. The present teachings are not limited to a disc or a cylinder, nor are the channels limited to a diamond shaped cross section. Moreover, the cross-sectional shape of one or more channels may differ from that of other channels. The geometry of the cylinder was chosen to illustrate the presenting teachings and is not intended to be limiting.

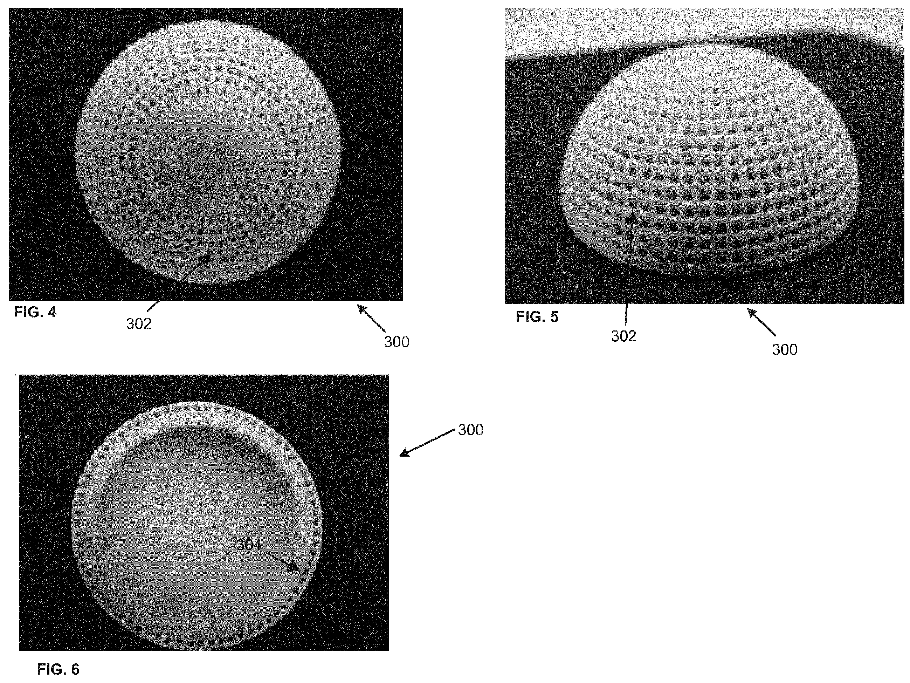

FIGS. 4-6 illustrate an acetabular cup 300 implant in accordance with one embodiment of the presenting teachings. A hip replacement consists of three parts: the acetabular cup, the femoral component, and the articular interface. The acetabular cup 300 comprises a hemispherical structure defining an interior cavity configured to receive the femoral head. The cup further has an outer convex surface that is configured to be received and fuse to bone. A problem with such cup implants is that the ingrowth or ongrowth may occur slowly or sporadically, leading to long recovery times or failure or such implants. The acetabular cup shown in FIGS. 4-6 is configured to encourage ingrowth of the bone.

FIG. 4 shows a top view of the cup 300. FIG. 5 shows a side view of the cup 300. FIG. 6 shows a bottom view of the cup. As shown in FIG. 6, the inner surface of the cup 300 is smooth. The acetabular cup 300 is constructed from PEKK using the SLS technique. The outer surface of the cup 300 (shown in FIGS. 4-5) comprises a plurality of circular channels 302 that extend radially inward. Below the channels, a plurality of circumferentially extending reservoirs 304 extend through the cup and provide fluid communication with the plurality of circular channels. In reference to FIG. 6, the openings for the plurality of reservoir channels are illustrated. After the cup is surgically implanted, a fluid comprising a growth factor is periodically introduced into the reservoir channels via a pump or some other means through the openings of the reservoir channels or in some cases, through the openings of the circular channels. Such fluid flows through the reservoir channels to the outer surface of the cup 300 via the plurality of radially extending channels. The growth factor promotes osteogeneration between the bone and the implant, thereby decreasing recovery time and increasing the strength of the connection between the bone and the implant. It should be understood that the geometry of the outer surface, in and of itself, promotes ingrowth, and may be practiced in accordance with the presenting teachings without the fluid delivery system.

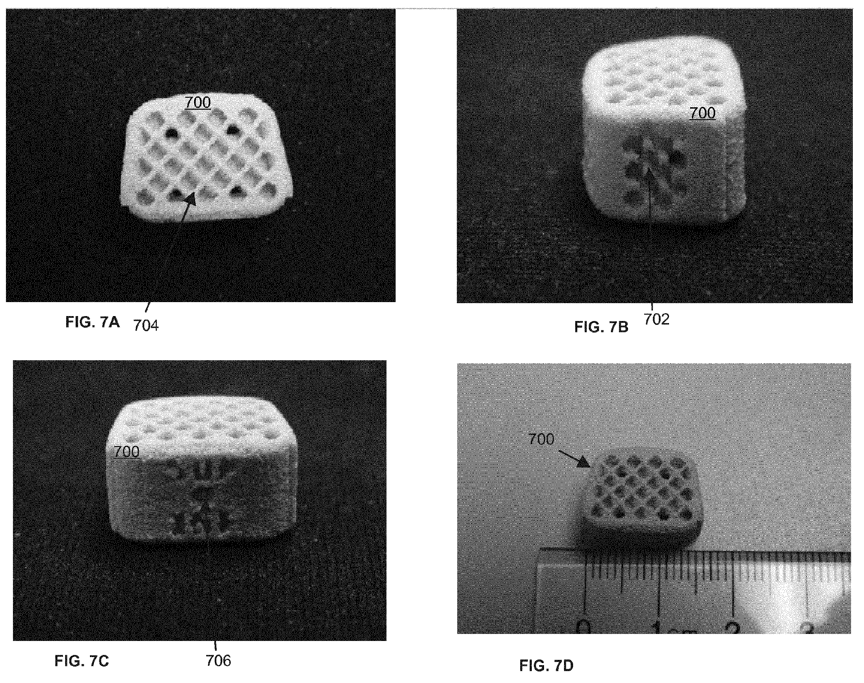

FIGS. 7A-7D illustrate a vertebral spacer 700 in accordance with one embodiment of the presenting teachings. A vertebral spacer is used in the spine to replace a collapsed, damaged, or unstable vertebral body. After the spacer is implanted into a patient, it is intended that surrounding bone will fuse to the spacer, thereby providing the patient with adequate support in the spine. There are several disadvantages for known implants. Particularly, the rate at which bone fuses to the vertebral spacer in some patients is insufficient and/or results in extended recovery periods. It has been discovered with respect to the present teachings that these disadvantages can be overcome by a vertebral spacer designed to encourage bone ingrowth and/or distribute therapeutic fluid after implantation.

FIG. 7A shows a top view of the vertebral spacer 700. FIG. 7B shows a side view of the spacer. FIG. 7C shows a side view of the spacer. FIG. 7D shows a top view of the spacer. The spacer 700 is made from PEKK using the above described SLS technique. The spacer defines an interior cavity 702 for receiving and/or retaining a therapeutic fluid. One or more of the surfaces of the spacer comprise a lattice structure 704. The lattice structure encourages post-operative osseointegration by ingrowth. Each lattice area is in fluid communication with the reservoir via or one or more channels. In this manner, it is possible to deliver a therapeutic fluid, such as a growth factor, to the interface between the bone and the spacer, thereby overcoming the disadvantages of the prior art. It should be understood that the lattice structure, in and of itself, promotes ingrowth, and may be practiced in accordance with the presenting teachings without the fluid delivery system.

In some embodiments, the spacer 700 is preloaded with sufficient therapeutic, such as a growth factor, to encourage bone growth without further post-operative recharging of the supply. In other embodiments of the presenting teachings, a port 706 is provided in the spacer such that it can be periodically recharged with therapeutic fluid. In yet other embodiments, a catheter or the like is provided and connected to the port. In some embodiments, the spacer is configured to deliver therapeutic fluid only to the extent needed. For example, a patient is post-operatively monitored. To the extent the patient's recovery fails to progress as predicted, hospital staff can intervene by activating a therapeutic delivery system, or adding therapeutic fluid to the spacer for delivery to the interface between the spacer and the bone. It should be understood to a person of ordinary skill in the art and familiar with this disclosure, that the spacer shown in FIGS. 7A-7D is intended to illustrate the presenting teachings, and is not intended to limit the invention. It should be understood that the implant may have different geometries depending on the patient and the intended use. It should further be understood, that the spacer may be custom fabricated for a specific patient. Finally, it should be understood that the form of the lattice structure and the reservoir may vary. In some embodiments, for example, the spacer does not include a lattice structure, but rather includes channels between the reservoir and the surface of the structure. The spacer according to such embodiments enhances ongrowth as opposed to ingrowth.

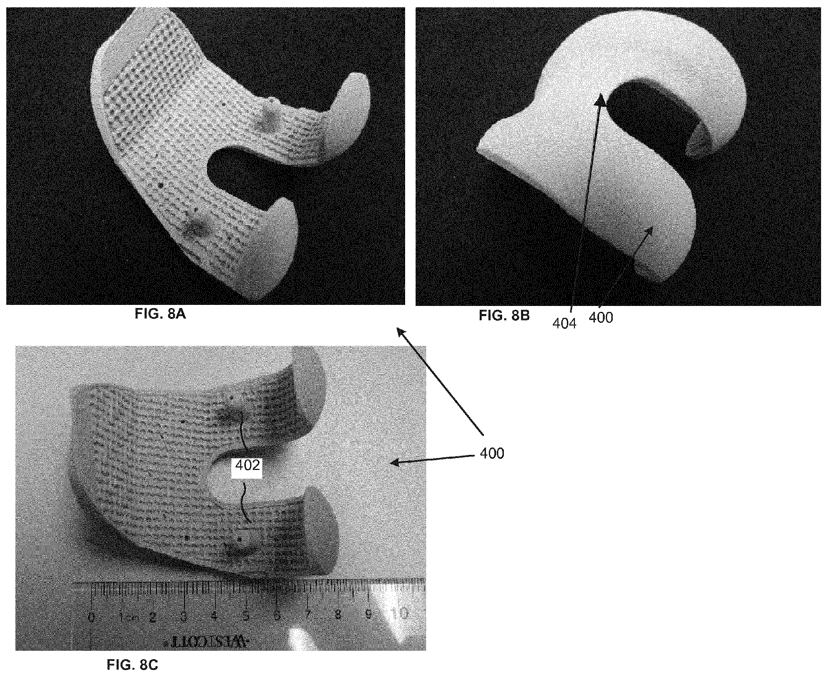

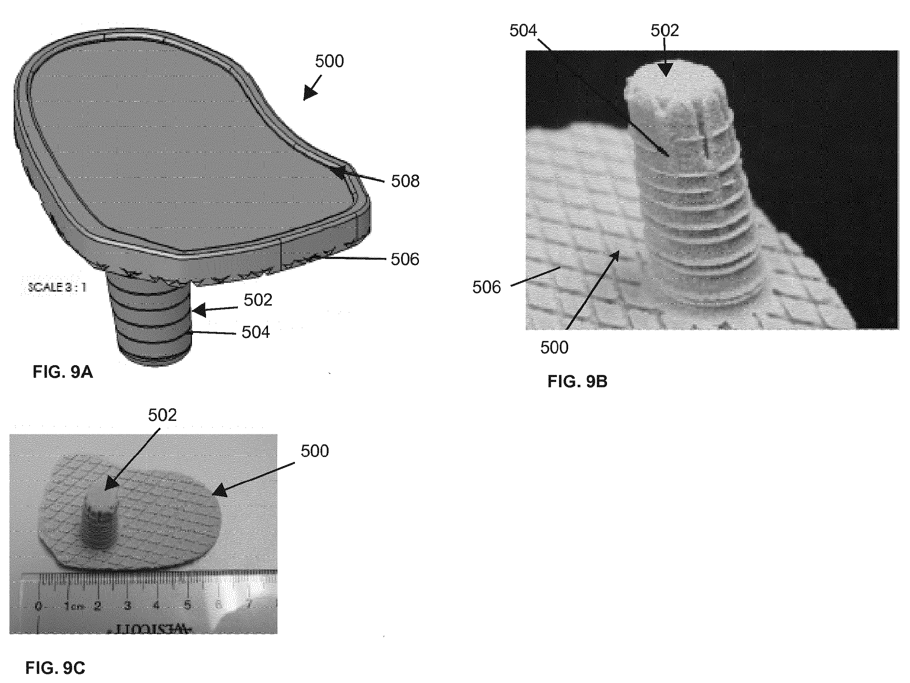

FIGS. 8A - 8C illustrate a femoral component 400 used in an artificial knee replacement, and FIGS. 9A-9C illustrate a tibial plateau 500 used in an artificial knee replacement. The femoral component 400 and the tibial plateau 500 are made from PEKK using the above described SLS technique. As a result, the components 400, 500 may be customized to a specific patient, thereby ensuring accuracy and further improving function (e.g., range of motion). The femoral component 400 includes one or more posts 402 that are configured to be received in the femur. A first side of the femoral component 400 configured to interface with the femur has a lattice like structural relief to encourage bone ingrowth, thereby facilitating recovery and overall success rates of implantation. In some embodiments of the presenting teachings, the femoral component may be fabricated with interior channels for delivery of a therapeutic fluid to the interface between the bone and the component. The opposing side 404 of the femoral replacement is designed to engage with a tibial plateau. This surface is smooth so as to facilitate a rotation of the surface of the femoral component 400 relative to the plateau during ambulation.

FIGS. 9A-9C illustrate a tibial plateau 500 used in an artificial knee replacement. The tibial plateau is a generally planar body. A first side includes one or more posts 502 configured to be received in and fuse with the tibia. In reference to FIG. 9B, a plurality of annular bars 504 are provided on the post 502 to facilitate retention of the post relative to the tibia and to facilitate fusion. The surface 506 of the plateau 500 from which the post 502 protrudes comprises a lattice like structural relief to encourage bone ingrowth, thereby facilitating recovery and overall success rates of implantation. In some embodiments of the presenting teachings, the tibial plateau may be fabricated with interior channels for providing delivery of a therapeutic fluid to the interface between the bone and the component. The opposing side 508 of the plateau 500 is designed to engage with a femoral component and is therefore relatively smooth.

### F. A LOAD BEARING KNEE SPACER FOR INHIBITING PERI-IMPLANT INFECTION BY ANTIBIOTIC PERFUSION.

One embodiment of the presenting teachings relates to an antibiotic eluding knee spacer which reduces the potential for total joint infection resulting from an artificial joint replacement and decreases the time of recovery from the joint replacement procedure. The current standard of care for chronic TJI is a two-stage revision. In the first stage, the infected implant is removed and the surrounding tissue is debrided. Next, an antibiotic (ABx) eluting polymer (poly methyl methacrylate, PMMA) spacer is implanted for 6 weeks during which time the patient is maintained on IV ABx. In the second stage, a new total joint replacement (TJR) is implanted. The entire treatment process requires a minimum of 2-3 months, followed by recovery from TJR. Success rates range from 63-100%.

There are a number of disadvantages associated with the current standard of care. First, there is currently a high bacterial resistance to the limited number of ABx that are compatible with PMMA. Second, given the 48-72 hour burst release of ABx from PMMA, the effective ABx levels fall off drastically. Third, it is not possible to vary the rate at which ABx is supplied or the type of ABx being supplied. Fourth, patients typically require systemic ABx, which can be very difficult for the body to handle.

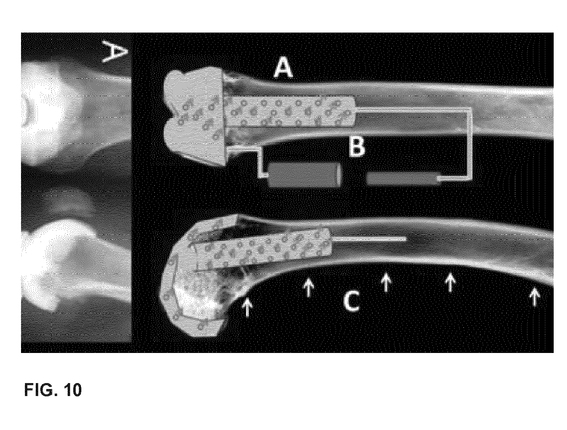

In an effort to overcome these problems associated with the prior art, the inventors have developed new drug-eluding spacers in accordance with the presenting teachings that are fabricated from PEKK using the SLS technique and that are capable of sustained delivery of ABx over extended periods. In reference to FIG. 10, a simplified schematic to validate ABx delivery from a knee spacer model is illustrated. The knee spacer may be fabricated from PEKK using the above described SLS technique. The spacer comprises a plurality of channels in fluid communication with a fluid source outside the body. The channels have openings at the surface of the spacer to elude ABx therefrom and affect the interface between the spacer and the tissue. It is preferable that such channels are not configured to open to the bearing surface of the spacer as delivery of fluid to the bearing surface may impact use thereof.

In this manner, the present invention enables the sustained delivery of ABx to an infected joint. Moreover, the delivery of antibiotics is specific to the site of infection, and therefore avoids the disadvantages associated with sustained administration of systemic ABx.

In one embodiment of the presenting teachings, the flow of fluid in the fluid communication system is reversible. For example, by applying a negative pressure to the fluid communication channel from outside of the body, it is possible to retrieve bodily fluid from around the knee. The retrieved fluid can be analyzed to determine the presence of infection and evaluate the effectiveness/efficacy of the antibiotic treatment. This innovation is advantageous because, for the first time, it enables ready access and analysis to the site of infection in TJI. If necessary, the treating personnel can adjust the amount or type of antibiotics being analyzed. Although this reverse fluid configuration is described in reference to the knee spacer, it is not limited in this regard, and may be used with other embodiments of the present teachings, including those specifically described herein.

### G. METHODS FOR FORMING APERTURES IN SELECTIVELY SINTERED COMPONENTS.

Another embodiment of the presenting teachings relates to methods of fabricating structures using SLS techniques, and more specifically methods for forming openings in the surface of a fabricated component. In the above described SLS processing, in which parts are formed from PEKK particles, it is difficult to consistently form features, for example openings, that are less that 0.5 mm in size. In some embodiments of the present teachings, the fluid communication system of an implant requires surface openings that are within this lower range to effectively control the flow of therapeutic fluid therethrough.

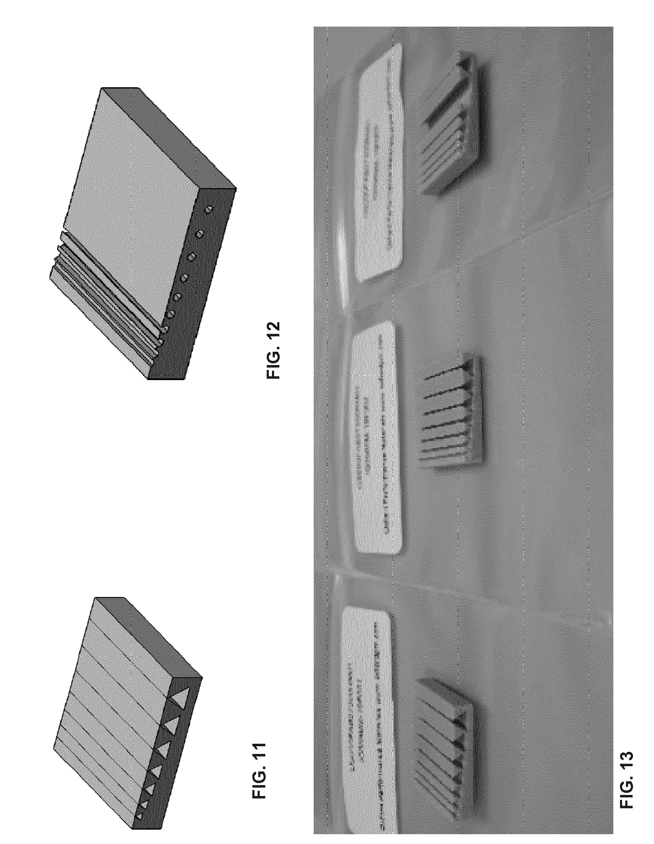

In order to overcome this limitation in the SLS technique, it has been discovered in the present teachings a method of forming apertures in the surface of parts (e.g., implant, implant components) made by the SLS technique. In accordance with this method, a subsurface cavity is formed in the part. For example, in reference to FIG. 11, a block having a plurality of cavities with triangular cross sections is illustrated. At least one of the angles of each triangle is proximate to (just below) the surface of the structure. This block or part is built using the SLS fabrication technique. Afterward, the built part is subjected to micro bead blasting, a technique that uses a stream of highly focused air comprising micro beads. As the micro beads contact the surface of the structure, they slowly erode potions of the surface. By directing the blast of micro beads at the surface of the structure, the area between the subsurface cavity and the surface is eroded, thereby slowly opening an aperture that connects the external atmosphere with the internal cavity.

By varying the geometry of the subsurface cavity, varying the distance between the surface of the part and a portion of the cavity, and/or varying the application of micro beads, it is possible to consistently form apertures in the surface of the implant that have a diameter that is less than that which could be attained using the SLS process itself. In this manner, it is possible to build small area openings to control the flow rate of fluid into and/or out of the channel.

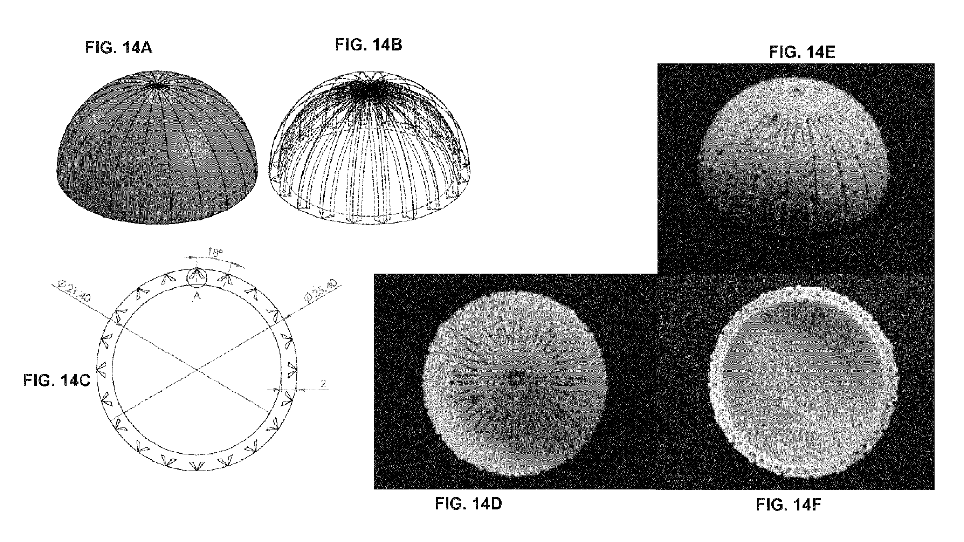

It should be understood that the geometry to practice this inventive method may vary depending on different factors, including the material and how the SLS technique is processed. In reference to FIG. 11 and FIG. 12, two test pieces are shown. The first including longitudinally extending subsurface cavities having triangular cross sections. The second comprising longitudinally extending subsurface cavities having circular cross sections. By varying the size of the cavities it is possible to determine the precise geometry to use to achieve apertures having a specific area. FIG. 13 shows three test blocks that have been subject to micro bead blasting, thereby creating the apertures therein. FIGS. 14A-14F illustrate this inventive technique being applied to the outside convex surface of an acetabular cup. The micro bead blasting could also be used to make small pores or chambers with small openings in the implant.

### H. ADDITIONAL EMBODIMENTS.

### i. SELECTIVELY SINTERED IMPLANT WITH THERAPEUTIC COATINGS.

In one embodiment of the presenting teachings, an implant made from the SLS process comprises an outer coating that is added after the SLS fabrication for providing controlled release of therapeutics. In one embodiment, the PEKK is coated with conformal layers of biocompatible polymers that contain incorporated drug molecules. In the second embodiment, nanoparticles are loaded with drugs and adhered to the PEKK material surface. The nanoparticles may include, but are not limited to drug-loaded, protein-loaded, and/or gene-loaded nanoparticles. In some embodiments, the nanoparticle surface is configured for selective interaction with materials. In some embodiments of the presenting teachings, the nanoparticles are loaded onto the PEKK material after implantation.

### ii. SELECTIVELY SINTERED IMPLANTABLE RIB.

In one embodiment of the presenting teachings, an artificial rib is printed using the SLS technique. PEKK is preferred in making the artificial rib as it is radiolucent, light, has mechanical properties similar to bone, can be cut and drilled like bone, and can be fabricated in complex geometries. Bone apposition to PEKK has been demonstrated and 3D surfaces that are suitable for soft tissue ingress and vascularization have been developed.

### iii. SELECTIVELY SINTERED IMPLANTABLE INSULIN PUMP.

In one embodiment, the present teachings relate to an implantable device for the delivery of insulin / glucagon made from PEKK. PEKK is a biocompatible polymer of high strength suitable for long term implantation. PEKK also provides exceptional electrical insulation, making it ideal as a substrate for printed circuits, such as circuits necessary to monitor blood glucose and release insulin as need.

### iv. SELECTIVELY SINTERED AUGMENTATION FOR GLENOID.

One embodiment of the presenting teachings relates to a patient-specific implant made from PEKK using the SLS technique for augmentation of lost or diseased bone to assist with reconstruction of the glenoid during shoulder revision. In another embodiment, the implant is configured for total shoulder arthroplasty.

### V. SELECTIVELY SINTERED SYSTEMS FOR LOWER EXTREMITY.

One embodiment of the presenting teachings relates to polymer SLS solutions for the treatment of sports, traumatic, arthritic, deformative, diabetic, and reconstructive pathology in the foot, ankle and other extremities. PEKK is radiolucent and light, has mechanical properties similar to bone, can be cut and drilled like bone, and can be fabricated in complex geometries. Bone apposition to PEKK has been demonstrated and 3D surfaces that are suitable for soft tissue ingress and vascularization have been developed. Current (prior art) implants weigh more than 10 times that of normal anatomy. Given the distance from center of mass seen in the extremities, PEKK's weight advantage may offer improved functional gait, biomechanics and biocompatibility.

Although the invention has been described with reference to a particular arrangement of parts, features and the like, these are not intended to exhaust all possible arrangements or features, and indeed many other modifications and variations will be ascertainable to those of skill in the art.

## Claims

1. A load-bearing implant, comprising:
a body with a shape fabricated using selective laser sintering, said body being made of Polyetherketoneketone (PEKK);
at least one reservoir forming an internal cavity inside said body, said reservoir having an opening that provides fluid access to said internal cavity from outside said body;
at least one fluid channel extending through said body between said at least one reservoir and an exterior surface of said body; and
a fluid stored in said reservoir, said fluid facilitating osseointegration between said body and a bone;
wherein said at least one fluid channel communicates said fluid from said reservoir to the exterior surface of said body and provides a controlled rate of delivery of said fluid through at least one outlet in the exterior surface of the body.

2. The load-bearing implant of claim 1, further comprising a plurality of fluid channels, each fluid channel having an outlet in the exterior surface of the body, wherein said outlets of said fluid channels are distributed along the entire exterior surface of the body to provide controlled delivery of said fluid at all points along said body.

3. The load-bearing implant of any one of the preceding claims, wherein said fluid includes at least one of an antibiotic solution, antifungal solution, protein factor, cells, or therapeutic solution.

4. The load-bearing implant of any one of the preceding claims, wherein said at least one fluid channel with said outlet is applied with negative pressure to draw bodily fluid surrounding the implant and to communicate said bodily fluid to said at least one reservoir, thereby said at least one fluid channel being configured to provide bidirectional fluid communication.

5. The load-bearing implant of any one of the preceding claims, further comprising an osmosis pump to transmit said at least one fluid through said at least one fluid channel, said osmosis pump being configured to connect to said opening of said reservoir or said at least one outlet.

6. The load-bearing implant of any one of the preceding claims, wherein at least a portion of the exterior surface of said body comprises a textured surface facilitating osseointegration between said body and a bone, said textured surface includes a lattice structure facilitating bone ingrowth.

7. The load-bearing implant of any one of the preceding claims, wherein at least a portion of the exterior surface of said body comprises a smooth bearing surface which facilitates movement of said implant relative to a complementary implant during ambulation.

8. The load-bearing implant of claim 7, wherein said at least one outlet and said at least one fluid channel are arranged in said body to release said fluid away from said smooth bearing surface.

9. The load-bearing implant of any one of the preceding claims, wherein said opening of said reservoir comprises a port disposed at the exterior surface of the body, said port regulating transmission of fluid through said opening for supplying fluid into said reservoir.

10. The load-bearing implant of claim 9, wherein said port is connectable via a catheter to an external supply of said fluid.

11. The load-bearing implant of claim 9, wherein said port is configured to receive a syringe or needle supplying said fluid to said reservoir.

12. The load-bearing implant of any one of the preceding claims, wherein said rate of delivery of said fluid is controlled by varying one or more of the following: a size of the at least one fluid channel, a size of the at least one outlet, a number of the at least one fluid channel, a viscosity of said fluid, a length of the at least one fluid channel, a cross-sectional shape of the at least one fluid channel, or a shape of the at least one outlet.

13. The load-bearing implant of any one of the preceding claims, further comprising at least one barb extending outwardly from the exterior surface of said body, said at least one barb anchoring said implant to a bone to minimize movement of said implant relative to said bone after implantation.

14. The load-bearing implant of any one of the preceding claims, further comprising a fluid supply unit connected to said reservoir, said fluid supply unit communicating said fluid to said reservoir through said opening, wherein said fluid supply unit is a bag implanted along with said implant.

15. The load-bearing implant of claim 14, further comprising a pump connectable to said implant to transmit said fluid from said reservoir through said at least one fluid channel to said at least one outlet in the exterior surface of the body, wherein said pump is implantable with said implant.
